# EUROPÄISCHE PATENTSCHRIFT

(11) **EP 0 825 234 B1**
(45) Veröffentlichungstag und Bekanntmachung des Hinweises auf die Patenterteilung: **30.05.2001**
(21) Anmeldenummer: 97810564.1
(22) Anmeldetag: 13.08.1997
(51) Int. Cl.: C09B 67/48, C09B 57/00, C08K 5/3415

(54) **Kristallmodifikation eines Diketopyrrolopyrrolpigments**
Crystal modification of a diketopyrrolopyrrole pigment
Modification cristalline d'un pigment de dicétopyrrolopyrrole

(30) Priorität: 22.08.1996 CH 206096
(43) Veröffentlichungstag der Anmeldung: 25.02.1998
(73) Patentinhaber: Ciba Specialty Chemicals Holding Inc., 4057 Basel (CH)
(72) Erfinder: Ruch, Thomas, 1723 Marly (CH); Wallquist, Olof, 1723 Marly (CH)

(56) Entgegenhaltungen:
- EP-A- 0 094 911
- EP-A- 0 408 498
- EP-A- 0 604 370
- EP-A- 0 648 770
- EP-A- 0 654 506
- EP-A- 0 690 059
- EP-A- 0 704 497
- EP-A- 0 748 851
- WO-A-90/01480

## Beschreibung

Die vorliegende Anmeldung betrifft eine neue Kristallmodifikation von 1,4-Diketo-3,6-diphenyl-pyrrolo[3,4-c]pyrrol, ihre Herstellung sowie die Verwendung dieses neuen Produktes als Pigment.

Es ist allgemein bekannt, dass eine Reihe von Vertretern verschiedener organischer Pigmentklassen, insbesondere bei Phthalocyanin-, Chinacridon- und einigen Azopig men-ten, polymorph sind, d.h. sie treten, trotz chemisch gleicher Zusammensetzung in zwei oder mehr Kristallmodifikationen auf. Im Falle der seit einigen Jahren bekannten und beispielsweise in den US-Patenten 4 415 685 und 4 579 949 beschriebenen Diketopyrrolopyrrolpigmente, sind bisher, wie aus US Patent 5591865, EP-A 690058 und EP-A 690059 zu entnehmen ist, nur für die drei Pigmente der Formeln 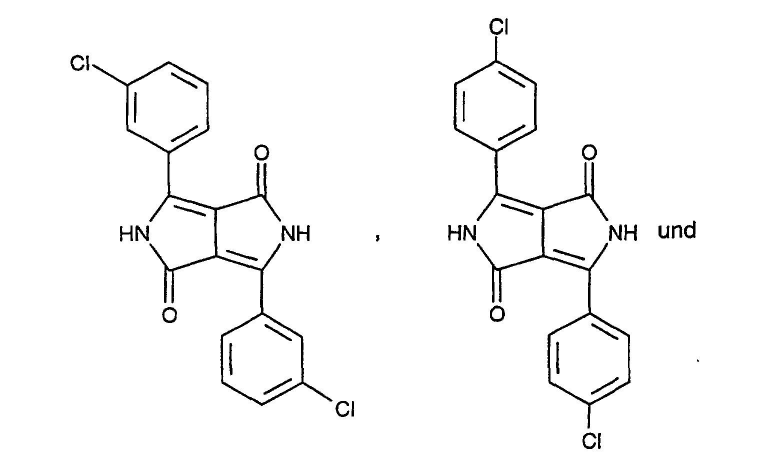 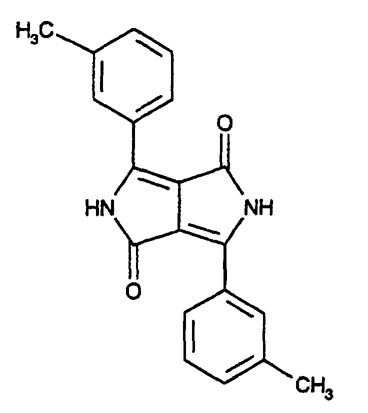 verschiedene Modifikationen bekannt. Sie werden durch Zurückführung in die Pigmentform aus einer löslichen latenten Pigmentform unter bestimmten Bedingungen erhalten.

Ferner werden in der EP-A-0 604 370 neue Kristallplättchen von 1,4-Diketo-3,6-diphenylpyrrolo[3,4-c]pyrrol beschrieben, die aus der bekannten Kristallform durch Umkristallisation aus aromatischen Lösungsmitteln bei Temperaturen über 220°C erhalten werden.

Es ist nun gefunden worden, dass durch Abänderung der in Beispiel 1 von US-Patent 4 579 949 beschriebenen Synthese des Pigments der Formel 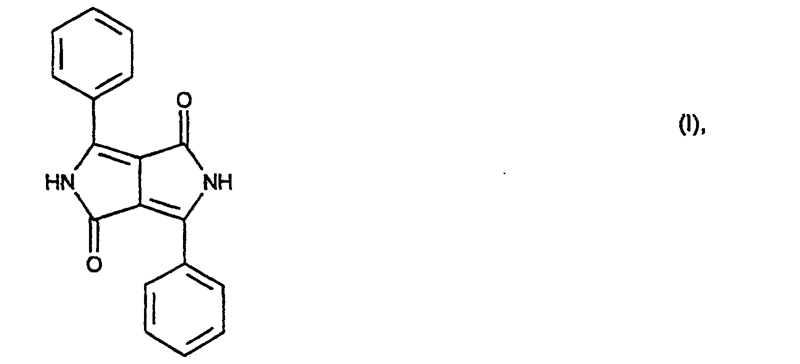 indem man vor oder während der Synthese eine kleine Menge eines cyansubstituierten Diketopyrrolopyrrols zugibt und nach der Protolyse das Protolysengemisch auf 50 bis 150°C erhitzt, ganz überraschend eine neue Kristallmodifikation des Pigments der Formel I erhalten wird. Die neue Modifikation im folgenden β-Modifikation genannt, unterscheidet sich von der bekannten Modifikation, im folgenden α-Modifikation genannt, durch ein bestimmtes, verschiedenes Röntgenbeugungsdiagramm, durch eine Nuancenverschiebung nach blaustichig Rot aber auch durch deutlich erhöhte Farbstärke und Sättigung sowie bessere Hitzebeständigkeit.

Die vollständigen Röntgenbeugungsdiagramme werden nach üblichen Methoden mit Hilfe eines Siemens D500® Röntgen-Diffraktometers (CuK_{α} -Strahlung) bestimmt.

Das Röntgenbeugungsdiagramm der bekannten α-Modifikation ist durch folgende Beugungslinien

| Netzbestände (d-Werte in Å) | doppelte Glanzwinkel (2 ⌀) | relative Intensität % |
|---|---|---|
| 13,9705 | 6,322 | 100 |
| 6,0698 | 14,582 | 49 |
| 5,8511 | 15,130 | 18 |
| 4,5886 | 19,328 | 18 |
| 3,8456 | 23,110 | 11 |
| 3,7449 | 23,740 | 23 |
| 3,3986 | 26,200 | 51 |
| 3,1710 | 28,118 | 33 |

gekennzeichnet

Die vorliegende Erfindung betrifft das Diketopyrrolopyrrol der Formel 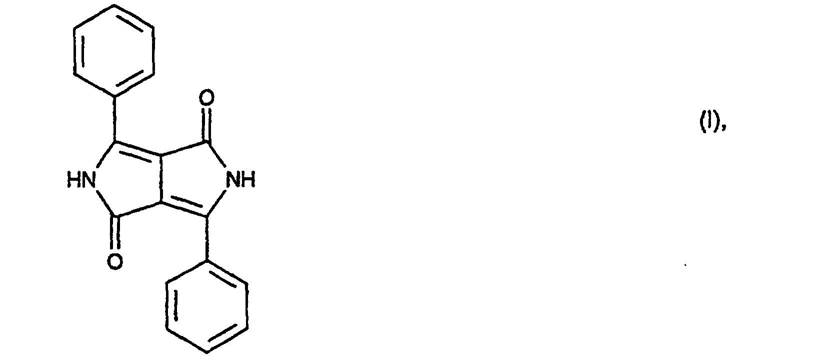 in seiner β-Modifikation, deren Röntgenbeugungsdiagramm durch folgende Beugungslinien

| Netzbestände (d-Werte in Å) | doppelte Glanzwinkel (2 ⌀) | relative Intensität % |
|---|---|---|
| 13,8817 | 6,362 | 100 |
| 4,9411 | 17,938 | 24 |
| 4,6743 | 18,971 | 26 |
| 4,5976 | 19,290 | 21 |
| 4,4193 | 20,076 | 11 |
| 3,3932 | 26,242 | 35 |
| 3,3195 | 26,836 | 33 |
| 3,0419 | 29,337 | 12 |

gekennzeichnet ist.

Die Herstellung dieser neuen β-Modifikation erfolgt durch Umsetzung von 1Mol eines Bernsteinsäuredialkylesters oder -diphenylesters, wobei im Bernsteinsäure esterrest Alkyl C₁-C₁₈-Alkyl und Phenyl unsubstituiertes oder durch ein oder zwei Halogenatome, eine oder zwei C₁-C₆-Alkyl- oder C₁-C₆-Alkoxygruppen substituiertes Phenyl bedeuten, mit 2 Mol Benzonitril in einem inerten organischen Lösungs mittel in Gegenwart eines Alkalimetalls oder eines Alkali-alkoholates als starke Base bei erhöhter Temperatur zu einem Pigmentalkalimetallsalz und anschliessende Freisetzung des Diketopyrrolopyrrols der Formel I durch Protolyse des entsprechenden Pig mentalkalimetallsalzes und nachfolgende Konditionierung nach allgemein bekannten Methoden,
dadurch gekennzeichnet, dass vor oder während dieser Synthese 2,2-20 Mol% eines Diketopyrrolopyrrols der Formel 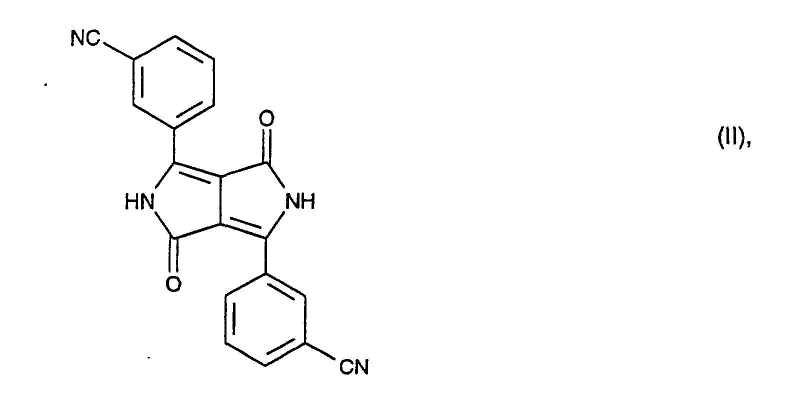 zugegeben werden und die Konditionierung bei einer Temperatur zwischen 50 und 120 °C durchgeführt wird.

C₁-C₁₈-Alkyl im Bernsteinsäureesterrest bedeutet z.B. Methyl, Ethyl, n-Propyl, Isopropyl, n-Butyl, tert.-Butyl, Amyl, Hexyl, Octyl, 2,2-Dimethylhexyl, Decyl, Dodecyl, Hexadecyl oder Octadecyl.

C₁-C₆-Alkyl und C₁-C₆-Alkoxy als Phenylsubstituenten im Bernsteinsäureesterrest bedeuten z.B. Methyl, Ethyl, n-Propyl, Isopropyl, n-Butyl, tert.-Butyl, Amyl, Hexyl, bzw. Methoxy, Ethoxy, n-Propoxy, Isopropoxy, n-Butoxy, Amyloxy und Hexyloxy.

Die Zugabe des Diketopyrrolopyrrols der Formel II erfolgt zweckmässig vor der Protolyse des Pigmentalkalisalzes oder bevorzugt am Anfang der Synthese, bevor Bern steinsäure-ester und Nitril zum vorgelegten Lösungsmittel und der Base gegeben werden. Bevorzugt werden 2,5-10 Mol% und insbesondere 2,5-6 Mol% des Diketopyrrolopyrrols der Formel II zugegeben.

Diketopyrrolopyrrole der Formel II sind bekannte Substanzen und können z.B. nach dem im US-Patent 4 579 949 beschriebenen Verfahren hergestellt werden. Die Protolyse erfolgt zweckmässig bei einem pH > 9, bevorzugt bei einem pH ≥ 11 und insbesondere bei einem pH > 12. Dabei kann zur Einstellung des pH's Säure vor, während oder nach der Protolyse zugegeben werden. Als Säuren können organische oder anorganische verwendet werden.

Die Konditionierung erfolgt vorzugsweise zwischen 50 und 80°C.

Auch das erfindungsgemässe β-Diketopyrrolopyrrol eignet sich, wie bereits z.B. in den US-Patenten 4415685 und 4579949 für dessen α-Modifikation beschrieben, als Pigment zum Färben von hochmolekularem organischem Material.

Wie viele andere Pigmente, kann das erfindungsgemässe β-Diketopyrrolopyrrol auch mit Vorteil nach bekannten Methoden oberflächenbehandelt werden, um seine Eigenschaften in Lacksystemen zu verbessern. Additive, die zur Verminderung oder Vermeidung von Flokkulation, sowie zur Verbesserung der Dispersionsstabilität dienen, können vorteilhaft mit dem erfindungsgemässen Pigment verwendet werden. Das so behandelte Pigment zeigt gute Eigenschaften allein oder in Mischung mit anderen Pigmenten zur Erzeugung von roten Volltonausfärbungen in verschiedenen Lacksystemen, jedoch bevorzugt in Automobillacksystemen des Acryl-, Alkyd- und Polyestertyps. 2-Phthalimidomethylchinacridon, Chinacridonsulfonsäure und andere ähnliche Derivate können z.B. als Antiflokkulationsmittel dienen. In bestimmten Systemen kann der Zusatz von polymeren Dispergiermittel die Eigenschaften des Pigments noch zusätzlich verbessern.

Das erfindungsgemässe β-Diketopyrrolopyrrol wird in Mengen von 0,01 bis 30 Gew.-%, bevorzugt 0,1 bis 10 Gew.-% bezogen auf das zu färbende hochmolekulare organische Material eingesetzt und in diesem zweckmässig bei Temperaturen zwischen 20 und 180°C eingearbeitet.

Das erfindungsgemässe β-Diketopyrrolopyrrol lässt sich beispielsweise als Pulver, Teig, Flushpaste und Zubereitung anwenden und eignet sich z.B. für Druckfarben, Leimfarben, Binderfarben oder Lacke aller Art, wie physikalisch und oxydativ trocknende Lacke, säure-, amin- und peroxid-härtende Lacke oder Polyurethanlacke. Das Pigment kann, dank seiner hohen Hitzebeständigkeit auch zum Färben von synthetischen, halbsynthetischen oder natürlichen makromolekularen Stoffen, selbst solchen, die bei hohen Temperaturen verarbeitet werden, verwendet werden, eventuell zusammen mit anderen organischen oder anorganischen Pigmenten. Die erhaltenen Färbungen, beispielsweise in Lacken, Drucken oder Kunststoffen, zeichnen sich durch einen blaustichig roten Farbton, gute Überlackier-, Migrations-, Licht- und Wetterechtheit, guter Hitzebeständigkeit sowie insbesondere durch hohe Farbstärke und Sättigung aus.

Das erfindungsgemässe Pigment kann zum Färben von festen, elastischen, pastenartigen, dickflüssigen, dünnflüssigen oder thixotropen Massen verwendet und in diese nach an sich bekannten Verfahren eingearbeitet werden. Wasser haltige Teige können beispielsweise durch Einrühren des Pigments in Wasser, gegebenenfalls unter Zusatz eines Netz- oder Dispergiermittels oder durch Einrühren oder Einkneten des Pigments i n ein Dispergiermittel in Gegenwart von Wasser und gegebenenfalls von organischen Lösungsmitteln oder Ölen erhalten werden. Diese Teige können beispielsweise wiederum zur Herstellung von Flushpasteten, Druckfarben, Leimfarben und Kunst stoffdispersionen verwendet werden. Das Pigment kann aber auch durch Einrühren, Einwalzen, Einkneten oder Einmahlen in Wasser, organische Lösungsmittel, nicht trocknende Öle, trocknen de Öle, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Die nachfolgenden Beispiele erläutern die Erfindung.

Beispiel 1 : 23,0 g Natrium werden zu 150 ml trockenem tert.-Amylalkohol gegeben, auf 100°C geheizt und gerührt bis das Natrium abreagiert hat. Die Lösung wird auf 100 °C gekühlt, dann werden zuerst 2,3 g (6,7 mMol) des Diketopyrrolopyrrols der Formel II, dann 31,25 g (99%ig, 0,3 Mol) Benzonitril und 50 ml tert.-Amylalkohol, und anschliessend.39,4 g Bernsteinsäurediisopropylester während 11 Stunden zugegeben. Am Ende der Zugabe wird mit 40 ml tert.-Amylalkohol verdünnt und für 2 Stunden bei 95 °C nachgerührt. Dann wird das Reaktionsgemisch warm zu einem Gemisch aus 170 ml Wasser und 170 ml Methanol bei einer Anfangstemperatur von 28 °C zugegeben. Nach Beendigung der Zugabe wird das Gemisch während 5 Stunden bei 45-48 °C gerührt, dann über Nacht auf 70 °C erwärmt und anschliessend filtriert. Der Rückstand wird mit Methanol und Wasser gewaschen bis das Filtrat farblos ist und dann in einem Trockenschrank bei 80 °C getrocknet. Man erhält 27,76 g eines roten Produktes, das in PVC eine blaustichig rote, transparente Färbung ergibt.

| Analyse : | C | H | N |
|---|---|---|---|
| Ber.: | 75.0 % | 4.2 % | 9.7 % |
| Gef.: | 74.8 % | 4.2 % | 9.8 % |

Das Röntgenbeugungsdiagramm ist durch folgende Beugungslinien

| Netzbestände (d-Werte in Å) | doppelte Glanzwinkel (2 ⌀) | relative Intensität % |
|---|---|---|
| 13,8817 | 6,362 | 100 |
| 4,9411 | 17,938 | 24 |
| 4,6743 | 18,971 | 26 |
| 4,5976 | 19,290 | 21 |
| 4,4193 | 20,076 | 11 |
| 3,3932 | 26,242 | 35 |
| 3,3195 | 26,836 | 33 |
| 3,0419 | 29,337 | 12 |

gekennzeichnet.

Beispiel 2: 23,0 g Natrium werden zu 150 ml trockenem tert.-Amylalkohol gegeben, auf 100°C geheizt und gerührt bis das Natrium abreagiert hat. Die Lösung wird auf 100 °C gekühlt, dann werden zuerst 1,52 g (4,5 mMol) des Diketopyrrolopyrrols der Formel II, dann 31,25 g (99%ig, 0,3 Mol) Benzonitril und 10 ml tert.-Amylalkohol, und anschliessend 39,4 g Bernsteinsäurediisopropylester während 5 Stunden zugegeben. Am Ende der Zugabe wird während 1 Stunde bei 100°C nachgerührt. Dann wird das Reaktionsgemisch zu einem Gemisch aus 170 ml Wasser und 170 ml Methanol zugegeben. Gleichzeitig wird konzentrierte Schwefelsäure dazugegeben, um den pH bei 12,3 zu halten. Nach Beendigung der Zugabe wird das Gemisch während 1 Stunde bei 66 °C und dann 6 Stunden auf Rückfluss erwärmt und anschliessend filtriert. Der Rückstand wird mit Methanol und Wasser gewaschen bis das Filtrat farblos ist und dann in einem Trockenschrank bei 80°C getrocknet. Man erhält 33,88 g eines roten Produktes, das gegenüber Beispiel 1 etwas blaustichiger ist.

| Analyse : | C | H | N |
|---|---|---|---|
| Ber.: | 75.0 % | 4.2 % | 9.7 % |
| Gef.: | 74.4 % | 4.3 % | 10.1 % |

Beispiel 3: Verfahren nach obigen Beispiel 2, bei dem 1,27 g (3,8 mMol) des Diketopyrrolopyrrols der Formel II eingesetzt wird. Erhalten wird 31,1 g eines roten

Pigments, das in PVC eine rote, transparente Färbung ergibt.

| Analyse : | C | H | N |
|---|---|---|---|
| Ber.: | 75.0 % | 4.2 % | 9.7 % |
| Gef.: | 74.3 % | 4.3 % | 9.9 % |

Beispiel 4: Verfahren nach obigen Beispiel 1, bei dem 5 g (15 mMol) des Diketopyrrolopyrrols der Formel II eingesetzt wird. Erhalten wird 27,14 g eines roten Pigments, das in PVC eine rote, transparente Färbung ergibt.

| Analyse : | C | H | N |
|---|---|---|---|
| Ber.: | 75.0 % | 4.2 % | 9.7 % |
| Gef.: | 73.9 % | 4.3 % | 9.9 % |

Beispiel 5: 18,4 g Natrium werden zu 200 ml trockenem tert.-Amylalkohol gegeben, auf 100°C geheizt und gerührt bis das Natrium abreagiert hat. Die Lösung wird auf 105 °C gekühlt, dann werden zuerst 1,8 g (5,4 mMol) des Diketopyrrolopyrrols der Formel II, dann 41,7 g (99%ig, 0,4 Mol) Benzonitril und 10 ml tert.-Amylalkohol, und anschliessend 52,6 g Bernsteinsäurediisopropylester während 5 Stunden zugegeben. Am Ende der Zugabe wird während 1 Stunde bei 104°C nachgerührt. Dann wird das Reaktionsgemisch warm zu einem Gemisch aus 170 ml Wasser und 170 ml Methanol bei einer Anfangstemperatur von 50°C zugegeben. Nach Beendigung der Zugabe wird das Gemisch während 1 Stunde bei 50°C, dann während 2 Stunden auf 60°C erwärmt und anschliessend filtriert. Der Rückstand wird mit Methanol und Wasser gewaschen bis das Filtrat farblos ist und dann in einem Trockenschrank bei 80°C getrocknet. Man erhält 42 g eines roten Produktes, das in PVC eine rote, transparente Färbung ergibt.

Beispiel 6: 7,5 g des Pigments, dessen Herstellung in Beispiel 1 beschrieben wird, 98,9 g CAB-Lösung bestehend aus

| | |
|---|---|
| 41,00 g | Celluloseacetobutyrat ®CAB 531.1, 20%ig in Butanol/Xylol 2:1 (Eastman Chem.) |
| 1,50 g | Zirkonium Octoat |
| 18,50 g | ®SOLVESSO 150∗ |
| 21,50 g | Butylacetat und |
| 17,50 g | Xylol |

| | |
|---|---|
| ∗ ®SOLVESSO 150 | |

36,50 g Polyesterharz ® DINAPOL H700 (Dynamit Nobel), 4,60 g Melaminharz ® MAPRENAL MF650 (Hoechst) und 2,50 g Dispergiermittel ® DISPERBYK 160 (BykChemie) werden zusammen während 90 Minuten mit einer Schüttelmaschine dispergiert (Total Lack 150g, 5% Pigment).
27,69 g des so erhaltenen Volltonlacks werden für die Base-coat-Lackierung mit 17,31 g Al-Stammlösung (8 %ig) bestehend aus

| | |
|---|---|
| 12,65 g | ®SILBERLINE SS 3334AR, 60 %ig (Silberline Ltd.) |
| 56,33 g | CAB Lösung (Zusammensetzung wie oben) |
| 20,81 g | Polyesterharz ®DINAPOL H700 |
| 2,60 g | Melaminharz ®MAPRENAL MF650 |
| 7,59 g | ®SOLVESSO 150∗ |

| | |
|---|---|
| ∗ ®SOLVESSO 150 | |

gemischt und auf ein Aluminiumblech spritzappliziert (Nassfilm ca.20 µm). Nach einer Abdunstzeit von 30 Minuten bei Raumtemperatur wird ein TSA-Lack bestehend aus

| | |
|---|---|
| 29,60 g | Acrylharz ® URACRON 2263 XB, 50 %ig in Xylol/Butanol (Chem. Fabrik Schweizerhalle), |
| 5,80 g | Melaminharz ® CYMEL 327, 90 %ig in Isobutanol, |
| 2,75 g | Butylglycolacetat, |
| 5,70 g | Xylol, |
| 1,65g | n-Butanol, |
| 0,50 g | Siliconöl, 1 %ig in Xylol, |
| 3,00 g | Lichtschutzmittel ® TINUVIN 900, 10 %ig in Xylol (Ciba), |
| 1,00 g | Lichtschutzmittel ® TINUVIN 292, 10 %ig in Xylol (Ciba) |

als Top-coat-Lackierung spritzappliziert (Nassfilm ca. 50 µm). Anschliessend wird der Lack nach weiteren 30 Minuten Abdunsten bei Raumtemperatur, 30 Minuten bei 130°C eingebrannt.

Beispiel 7: 0,6g des gemäss Beispiel 1 hergestellten Pigments werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte rote PVC-Folie zeichnet sich durch sehr gute Echtheiten aus.

### * ® SOLVESSO 150

Beispiel 8: Eine Mischung von 1,0 g des nach Beispiel 1 erhaltenen Pigmentes, 1,0 g Antioxidans (IRGANOX® 1010, CIBA-GEIGY AG) und 1000 g Polyethylen-HD Granulat (® VESTOLEN 60-16, HUELS) wird während 15 Minuten in einer Glasflasche auf einer Rollbank vorgemischt. Danach wird die Mischung in zwei Passagen auf einem Einwellenextruder extrudiert, das so erhaltene Granulat wird auf der Spritzgussmaschine (Ferromatik Aarburg 200) in Portionen je 5 Minuten bei 200, 220, 240, 260, 280 und 300°C zu Platten verspritzt. Alle Pressplatten weisen blaustichig rote Nuancen mit guten Beständigkeiten auf.

## Patentansprüche

1. Diketopyrrolopyrrol der Formel 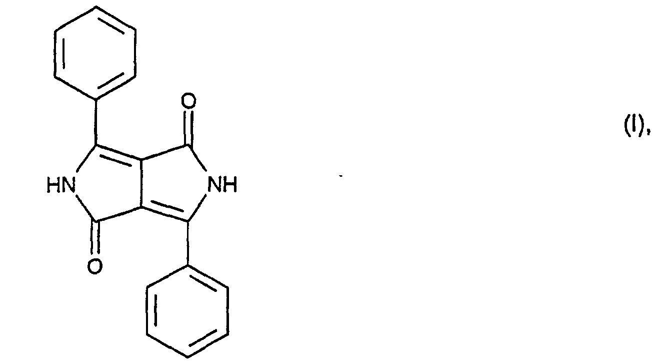
in seiner β-Modifikation, deren Röntgenbeugungsdiagramm durch folgende Beugungslinien
| Netzbestände (d-Werte in Å) | doppelte Glanzwinkel (2 ⌀) | relative Intensität % |
|---|---|---|
| 13,8817 | 6,362 | 100 |
| 4,9411 | 17,938 | 24 |
| 4,6743 | 18,971 | 26 |
| 4,5976 | 19,290 | 21 |
| 4,4193 | 20,076 | 11 |
| 3,3932 | 26,242 | 35 |
| 3,3195 | 26,836 | 33 |
| 3,0419 | 29,337 | 12 |
gekennzeichnet ist.

2. Verfahren zur Herstellung des Diketopyrrolopyrrols gemäss Anspruch 1 durch Umsetzung von 1Mol eines Bernsteinsäuredialkylesters oder -diphenylesters, wobei im Bernsteinsäureesterrest Alkyl C₁-C₁₈-Alkyl und Phenyl unsubstituiertes oder durch ein oder zwei Halogenatome, eine oder zwei C₁-C₆-Alkyl- oder C₁-C₆-Alkoxygruppen substituiertes Phenyl bedeuten, mit 2 Mol Benzonitril in einem inerten organischen Lösungs mittel in Gegenwart eines Alkalimetalls oder eines Alkali-alkoholates als starke Base bei erhöhter Temperatur zu einem Pigmentalkalimetallsalz und anschliessende Frei setzung des Diketopyrrolopyrrols der Formel I durch Protolyse des entsprechenden Pig mentalkalimetallsalzes und nachfolgende Konditionierung nach allgemein bekannten Methoden,
dadurch gekennzeichnet, dass vor oder während dieser Synthese 2,2-20 Mol% eines Diketopyrrolopyrrols der Formel 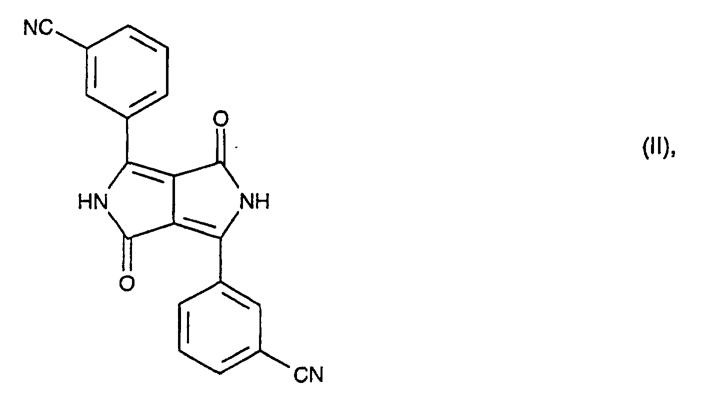 zugegeben werden und die Konditionierung bei einer Temperatur zwischen 50 und 120 °C und einem pH >9 durchgeführt wird.

3. Verfahren gemäss Anspruch 2, dadurch gekennzeichnet, dass das Diketopyrrolopyrrol der Formel II vor der Protolyse des Pigmentalkalisalzes zugegeben wird.

4. Verfahren gemäss Anspruch 3, dadurch gekennzeichnet, da ss das Diketopyrrolopyrrol der Formel II am Anfang der Synthese zugegeben wird.

5. Verfahren gemäss Anspruch 2, dadurch gekennzeichnet, dass das Diketopyrrolopyrrol der Formel II in einer Menge von 2,5-10 Mol% zugegeben wird.

6. Verfahren gemäss Anspruch 5, dadurch gekennzeichnet, dass das Diketopyrrolopyrrol der Formel II in einer Menge von 2,5-6 Mol% zugegeben wird.

7. Verfahren gemäss Anspruch 2, dadurch gekennzeichnet, dass die Konditionierung bei einem pH ≥ 11 durchgeführt wird.

8. Verfahren gemäss Anspruch 2, dadurch gekennzeichnet, dass die Konditionierung zwischen 50 und 80°C durchgeführt wird.

9. Hochmolekulares organisches Material enthaltend ein Diketopyrrolopyrrol gemäss Anspruch 1.

## Claims

1. A diketopyrrolopyrrole of formula 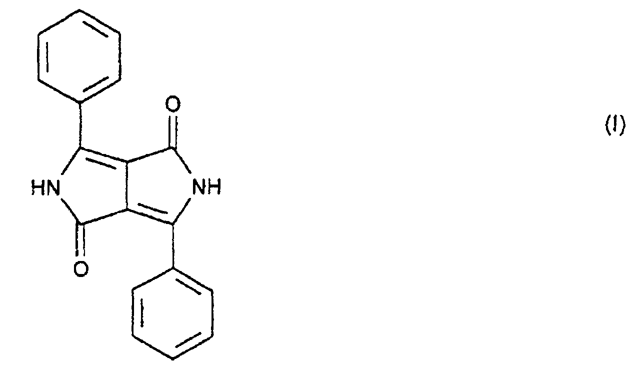 in its β-modification, the X-ray diffraction pattern of which is characterised by the following diffraction lines:
| interplanar spacings | double glancing angles | relative intensity |
|---|---|---|
| (d values in Å) | (2 Ø) | % |
| 13.8817 | 6.362 | 100 |
| 4.9411 | 17.938 | 24 |
| 4.6743 | 18.971 | 26 |
| 4.5976 | 19.290 | 21 |
| 4.4193 | 20.076 | 11 |
| 3.3932 | 26.242 | 35 |
| 3.3195 | 26.836 | 33 |
| 3.0419 | 29.337 | 12 |

2. A process for the preparation of the diketopyrrolopyrrole according to claim 1 by reacting 1 mol of a dialkyl succinate or diphenyl succinate, where the alkyl in the succinate moiety is C₁-C₁₈alkyl and the phenyl is unsubstituted or substituted by one or two halogen atoms, one or two C₁-C₆alkyl groups or C₁-C₆alkoxy groups, with 2 mol of benzonitrile in an inert organic solvent in the presence of an alkali metal or alkali metal alcoholate as a strong base at elevated temperature to give a pigment alkali metal salt which is subsequently protolysed to liberate the diketopyrrolopyrrole of formula I and then conditioned by commonly known methods,
which comprises adding before or during this synthesis 2.2-20 mol% of a diketopyrrolopyrrole of formula 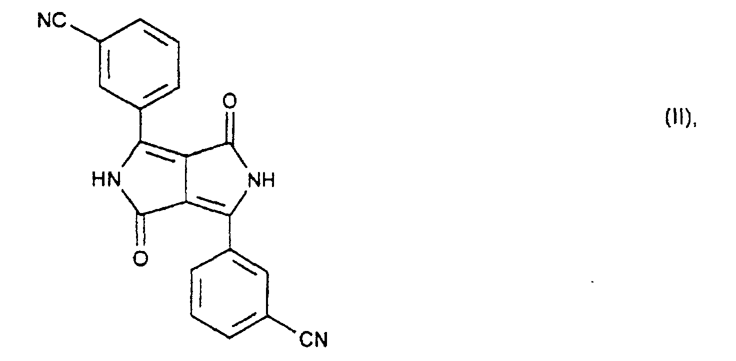 and carrying out the conditioning at a temperature between 50 and 120°C and at a pH > 9.

3. A process according to claim 2, which comprises adding the diketopyrrolopyrrole of formula II prior to the protolysis of the pigment alkali metal salt.

4. A process according to claim 3, which comprises adding the diketopyrrolopyrrole of formula II at the start of the synthesis.

5. A process according to claim 2, which comprises adding the diketopyrrolopyrrole of formula II in an amount of 2.5-10 mol%.

6. A process according to claim 5, which comprises adding the diketopyrrolopyrrole of formula II in an amount of 2.5-6 mol%.

7. A process according to claim 2, which comprises carrying out the conditioning at a pH ≥ 11.

8. A process according to claim 2, which comprises carrying out the conditioning at between 50 and 80°C.

9. A high molecular weight organic material comprising a diketopyrrolopyrrole according to claim 1.

## Revendications

1. Dicétopyrrolopyrrole de formule 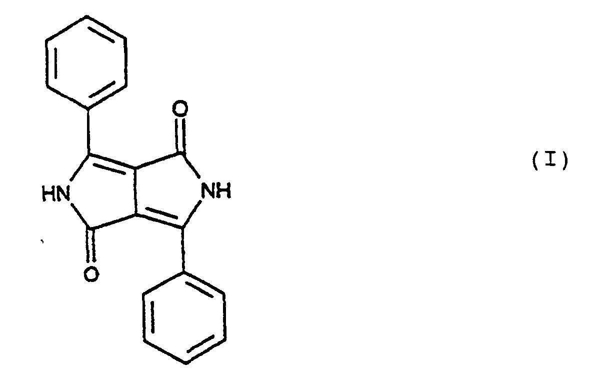 dans sa modification β, dont le diagramme de diffraction des rayons X est caractérisé par les lignes diffractées suivantes
| Ecartement des plans réticulaires (valeurs d en Å) | double angle de brillance (2 θ) | Intensité relative % |
|---|---|---|
| 13,8817 | 6,362 | 100 |
| 4,9411 | 17,938 | 24 |
| 4,6743 | 18,971 | 26 |
| 4,5976 | 19,290 | 21 |
| 4,4193 | 20,076 | 11 |
| 3,3932 | 26,242 | 35 |
| 3,3195 | 26,836 | 33 |
| 3,0419 | 29,337 | 12 |

2. Procédé pour la préparation du dicétopyrrolopyrrole selon la revendication 1 par réaction de 1 mole d'un ester dialkylique ou diphénylique de l'acide succinique, le groupe alkyle dans le reste ester de l'acide succinique représente un alkyle en C₁-C₁₈ et le groupe phényle représente un phényle non substitué ou substitué par un ou deux atomes d'halogènes, un ou deux groupes alkyle en C₁-C₆ et ou alkoxy en en C₁-C₆, sur 2 moles de benzonitrile dans un solvant organique inerte en présence d'un métal alcalin ou d'un alcoolate alcalin en tant que base forte, à température élevée, pour obtenir un sel de pigment de métal alcalin et par libération ultérieure du dicétopyrrolopyrrole de formule I par protolyse du sel de pigment de métal alcalin et par conditionnement ultérieur selon des méthodes connues,
caractérisé en ce qu'on ajoute avant ou au cours de la synthèse de 2,2 à 20% molaires d'un dicétopyrrolopyrrole de formule 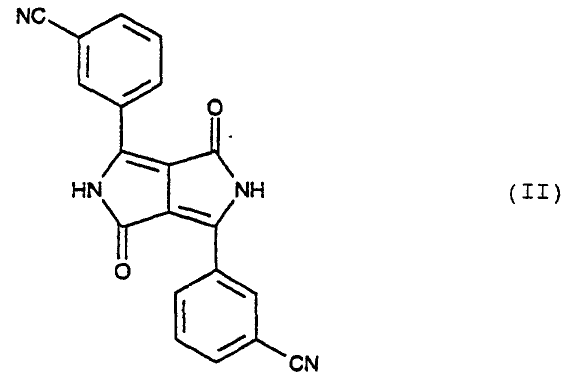 et on met en oeuvre le conditionnement à une température comprise entre 50 et 120°C et un pH >9.

3. Procédé selon la revendication 2,
caractérisé en ce qu'on ajoute le dicétopyrrolopyrrole de formule II avant la protolyse du sel alcalin du pigment.

4. Procédé selon la revendication 3,
caractérisé en ce qu'on ajoute le dicétopyrrolopyrrole de formule II au début de la synthèse.

5. Procédé selon la revendication 2,
caractérisé en ce qu'on ajoute le dicétopyrrolopyrrole de formule II en une quantité de 2,5 à 10% molaires.

6. Procédé selon la revendication 5,
caractérisé en ce qu'on ajoute le dicétopyrrolopyrrole de formule II en une quantité de 2,5 à 6% molaires.

7. Procédé selon la revendication 2,
caractérisé en ce qu'on met en oeuvre le conditionnement à un pH ≥ 11.

8. Procédé selon la revendication 2,
caractérisé en ce qu'on oeuvre le conditionnement à une température comprise entre 50 et 80°C.

9. Matière organique de haut poids moléculaire contenant un dicétopyrrolopyrrole selon la revendication 1.
